# EUROPÄISCHE PATENTSCHRIFT

(11) **EP 1 854 647 B1**
(45) Veröffentlichungstag und Bekanntmachung des Hinweises auf die Patenterteilung: **10.11.2010**
(21) Anmeldenummer: 07009533.6
(22) Anmeldetag: 11.05.2007
(51) Int. Cl.: B60H 3/00

(54) **Beduftungssystem, insbesondere für ein Kraftfahrzeug**
Fragrance system, in particular for a motor vehicle
Système de parfum, en particulier pour un véhicule automobile

(30) Priorität: 12.05.2006 DE 102006022661
(43) Veröffentlichungstag der Anmeldung: 14.11.2007
(73) Patentinhaber: Behr GmbH & Co. KG, 70469 Stuttgart (DE)
(72) Erfinder: Lochmahr, Karl, 71665 Vaihingen/Enz (DE); Burr, Reinhold, 89522 Heidenheim (DE); Stiehler, Daniela, 70597 Stuttgart (DE)

(56) Entgegenhaltungen:
- EP-A- 1 561 477
- EP-A1- 1 398 187
- WO-A-03/049961
- GB-A- 2 249 958
- US-A- 5 314 669
- US-A- 5 429 180

## Beschreibung

Die Erfindung betrifft ein Beduftungssystem gemäß dem Oberbegriff des Anspruches 1.

Aus der DE 103 27 122 A1 ist eine Belüftungsvorrichtung, insbesondere zum Beduften eines Kraftfahrzeugs, bekannt, welche einen Duftstoff enthält, mit einer Dosiereinrichtung, wobei die Beduftungsvorrichtung zumindest einen Hohlkörper, der mit mindestens einer Öffnung versehen ist, und ein Element, insbesondere einen Hohlkörper, mit mindestens einer weiteren Öffnung umfasst. Dabei ist der Hohlkörper und/oder das Element mittels eines Motors derart antreibbar, dass eine Relativbewegung zwischen dem Hohlkörper und dem Element möglich ist, so dass die beiden Öffnungen in zumindest einer Stellung der Relativbewegung ein Austreten von Duftstoff von innen nach außen ermöglichen. Der Duftstoff selbst ist innerhalb des Hohlkörpers in einer Duftstoffpatrone oder -kartusche angeordnet, die bei Bedarf auswechselbar ist.

Eine andere Beduftungsvorrichtung ist aus der US 5,314,669 A bekannt. Bei dieser Beduftungsvorrichtung sind zwei in axialer Richtung zueinander angeordnete Hohlzylinder in einem im Wesentlichen als Hohlzylinder ausgebildeten Gehäuse drehbar aufgenommen. Der das Gehäuse bildende Hohlzylinder weist zwei rechteckförmige, sich in Längsrichtung erstreckenden Öffnungen in seiner Mantelfläche auf einander gegenüberliegenden Seiten auf, die sich jeweils zumindest über einen Teilbereich der inneren Hohlkörper erstrecken. Die inneren Hohlzylinder weisen ebenfalls jeweils zwei einander gegenüberliegende Öffnungen in ihren Mantelflächen auf, die jedoch gestuft ausgebildet sind, d.h. die Form dreier jeweils um die Hälfte ihrer Länge versetzt zueinander angeordneter, langgestreckter und zusammen eine gemeinsame Öffnung bildender Rechtecke aufweisen. Die Öffnungen der inneren Hohlzylinder sind verdreht zueinander angeordnet, so dass verschiedene Beduftungsvariationen möglich sind.

Die US 5,178,327 A offenbart ein Beduftungssystem mit einem in mehrere Sektoren unterteilten, drehbar in einem Gehäuse angeordneten Hohlzylinder, wobei mindestens ein kuchenartiger Teil des Hohlzylinders in der Größe eines Segments fehlt, weshalb auf den Hohlzylinder im Folgenden als Teil-Hohlzylinder Bezug genommen wird. Die Segmente sind jeweils stirnseitig offen und in jedem der Segmente ist ein anderer Duftstoff eingelagert. Im Gehäuse ist auf einer oder beiden Seiten in axialer Richtung des Teil-Hohlzylinders eine in Ihrer Gestalt im Wesentlichen dem Querschnitt eines Segments entsprechende Öffnung ausgebildet. Um einen Raum zu beduften, wird das den gewünschten Duft enthaltende Segment vor die Öffnung gedreht. Wird keine Beduftung gewünscht, so wird das fehlende Segment vor die Öffnung gedreht. Um eine stärkere Beduftung eines Raumes zu ermöglichen, kann auch ein Lüfter vorgesehen sein, welches Luft durch die erste Öffnung in das Gehäuse und nach dem Durchströmen des Teil-Hohlzylinders durch die zweite, gegenüberliegende Öffnung dem zu beduftenden Raum zuführt.

Die GB 2 249 offenbart ein Beduftungssystem, welches in einem runden Gehäuse angeordnet ist und vier Sektoren aufweist, von denen drei mit Filzträgern, welche mit drei unterschiedlichen Düften imprägniert sind, aufweist.

Derartige Beduftungssysteme lassen noch Wünsche offen.

Es ist Aufgabe der Erfindung, ein verbessertes Beduftungssystem zur Verfügung zu stellen.

Diese Aufgabe wird gelöst durch ein Beduftungssystem mit den Merkmalen des Anspruchs 1. Vorteilhafte Ausgestaltungen sind Gegenstand der Unteransprüche.

Hierbei ist ein Beduftungssystem, insbesondere für ein Kraftfahrzeug, vorgesehen, mit zumindest einem ersten Bauteil und zumindest einem zweiten Bauteil, welches das zumindest erste Bauteil im Wesentlichen umgreift oder von diesem umgriffen wird, wobei das zumindest eine erste und das zumindest eine zweite Bauteil jeweils eine prismatische Innen- beziehungsweise Außenmantelfläche aufweisen, die auch unterbrochen ausgebildet sein kann, und die Bauteile relativ zueinander bewegbar sind, in einem der Bauteile mindestens zwei Aufnahmekammern vorgesehen sind, in welchen mindestens ein Duftstoff enthalten ist, und durch das zumindest eine, erste Bauteil und das zumindest eine, zweite Bauteil in Abhängigkeit der Position zueinander mindestens eine Verbindung zur Umgebung freigeben ist, über welche bei Bedarf Duftstoff nach außen aus der oder den Aufnahmekammern abgegeben werden kann. Dabei sind erfindungsgemäß die Aufnahmekammern durch zumindest eine in Längsrichtung des Bauteils verlaufende Trennwand voneinander getrennt sind, und die Verbindung zur Umgebung ist über zumindest eine in einer prismatischen Mantelfläche des Bauteils, in welchem die Aufnahmekammern für den oder die Duftstoffe angeordnet sind, ausgebildete Öffnung und mindestens eine Öffnung in der gegenüberliegend angeordneten Mantelfläche des anderen Bauteils, vorgesehen. Zustand mindestens eine Aufnahmekammer ohne einen Duftstoff vorgesehen ist. Ein derartiges System hat einen sehr einfachen Aufbau und lässt sich relativ einfach regeln, wobei neben den Beduftungszuständen bei entsprechender Ausgestaltung auch ein Zustand ohne Beduftung möglich ist, was insbesondere in Hinblick auf die allmähliche Gewöhnung an einen Duft bei konstanter Beduftung hilfreich ist. Die Öffnungen zu den einzelnen Aufnahmekammern, in denen ein oder kein Duftstoff enthalten ist, werden auf sehr einfache Weise durch eine (oder mehrere) Öffnungen im zweiten Bauteil freigegeben, so dass bedarfsgerecht die entsprechenden Öffnungen derart ausgerichtet werden können, damit eine direkte Verbindung vom Innenraum der entsprechenden Aufnahmekammer oder ggf. zweier Aufnahmekammern und der Umgebung, also bspw. dem Fahrzeuginnenraum, zur Verfügung gestellt werden kann.

Bevorzugt ist mindestens eine Mantelfläche eines Bauteils, die direkt gegenüberliegend des entsprechenden Mantelfläche des anderen Bauteils angeordnet ist, mit kreiszylindrischem Querschnitt ausgebildet. Insbesondere bevorzugt sind beide einander gegenüberliegende Mantelflächen von kreiszylindrischem Querschnitt.

Die Aufnahmekammern sind vorzugsweise in dem Bauteil angeordnet, welches vom anderen Bauteil umgriffen wird, wobei die Öffnungen der Aufnahmekammern in der Außenmantelfläche des Bauteils angeordnet sind.

Die Aufnahmekammern, in denen ein Duftstoff oder auch kein Duftstoff enthalten ist, sind vorzugsweise von sich in radialer Richtung erstreckenden Trennwänden unterteilt. Diese sind bevorzugt sternförmig in gleichen Winkeln zueinander angeordnet, insbesondere bevorzugt in 60°-, 90°- oder 120°-Winkeln.

Die Bauteile können relativ zueinander verdrehbar und/oder in Längsrichtung verschiebbar sein.

Bevorzugt sind die Aufnahmekammern über mindestens eine Öffnung, insbesondere bevorzugt über eine Mehrzahl von Bohrungen und/oder Schlitze, in der Außen- oder Innenmantelfläche des entsprechenden Bauteils vorgesehen sind, nach außen oder innen hin offen. Insbesondere Bohrungen oder Schlitze lassen sich besonders einfach ausbilden und können so dimensioniert werden, dass das Trägermaterial für den jeweiligen Duftstoff, bspw. ein Granulat, nicht durch die Öffnungen austreten kann. Gegebenenfalls kann jedoch der Duftstoff in einem weiteren, in der entsprechenden Aufnahmekammer angeordneten Behältnis, bspw. einer wiederbefüllbaren Patrone, angeordnet sein.

Das zweite, die Öffnungen des ersten Bauteils abdeckende Bauteil, weist vorzugsweise als eine Verbindung zur Umgebung bildende Öffnung mindestens einen Schlitz auf. Dieser Schlitz erstreckt sich bevorzugt über die gesamte Länge des äußeren Hohlzylinders oder Hohlkörpers. Er hat vorzugsweise eine Breite, die sich über maximal 120°, insbesondere bevorzugt über maximal 90°, des äußeren Hohlzylinders erstreckt. Besonders bevorzugt beträgt der Öffnungswinkel weniger als 60°, jedoch mehr als 10°, insbesondere mehr als 20°. Derartige Schlitzbreiten ermöglichen ein ausreichend gutes Ausströmen des Duftstoffs.

Besonders bevorzugt sind Dichtelemente in äquidistanten Abständen auf der Außenmantelfläche des inneren Bauteils oder der Innenmantelfläche des äußeren Bauteils in radialer Verlängerung von Trennwänden zwischen den einzelnen Aufnahmekammern angeordnet, d.h. sie verlaufen parallel zur Längsachse der konzentrisch angeordneten Bauteile. Die Dichtelemente sind bevorzugt elastisch ausgebildet und schleifen im Falle einer Stellbewegung eines der Bauteile an der gegenüberliegenden Mantelfläche, so dass ein unerwünschtes Aus- oder Überströmen der Duftstoffe verhindert werden kann.

Vorzugsweise sind mindestens zwei Duftstoffe vorgesehen, die in getrennten Aufnahmekammern angeordnet sind, und in mindestens einer weiteren Aufnahmekammer ist kein Duftstoff enthalten, insbesondere bevorzugt in jeweils einer zwischen zwei Duftstoffen enthaltenden Aufnahmekammer, so dass sich eine relativ einfache Regelung der Duftstoffabgabemenge durch der Stellung der beiden Bauteile zueinander und hiermit verbundener Teilüberdeckungen des Bereichs mit Öffnungen im ersten, den bzw. die Duftstoffe enthaltenden Bauteil ermöglicht.

Die einzelnen Aufnahmekammern weisen vorzugsweise gleiche Volumen auf. Dies vereinfacht die Montage und erleichtert eine Verwendung von entsprechend ausgebildeten Duftpatronen, die ggf. auswechselbar sind.

Die Bauteile sind erfindungsgemäß in einem Gehäuse angeordnet, in oder neben welchem ferner ein Lüfter zur Erzeugung eines Luftstroms vorbei an der mindestens einen Öffnung des Bauteils ohne Aufnahmekammern angeordnet ist. Dies ermöglicht eine einfache, ggf. auch nachträgliche Montage in einem Kraftfahrzeug, besonders bevorzugt zentral im Bereich der Instrumententafel. Die Anordnung des Beduftungssystems erfolgt vorzugsweise nicht in einem Ausströmer der Kraftfahrzeug-Klimaanlage, d.h. die Insassen werden bevorzugt nicht direkt vom Duftstoff angeströmt, was unter Umständen, insbesondere bei schnellen Konzentrationsänderungen, als unangenehm empfunden werden kann. Eine Integration in das Belüftungssystem ist ebenfalls möglich, so dass das Gebläse der Klimaanlage anstelle eines zusätzlichen Lüfters für das Fördern des entsprechenden (Teil-)Luftstroms verwendet werden kann.

Das Beduftungssystem wird vorzugsweise in einem Intervallbetrieb betrieben, bei dem einem ersten konstanten Zeitintervall mit Beduftung ein zweites konstantes Zeitintervall ohne Beduftung folgt.

Die Zeitspanne der Beduftung beträgt bevorzugt 5 bis 60 Sekunden, insbesondere bevorzugt ca. 10 Sekunden. Die Zeitspanne ohne Beduftung beträgt bevorzugt 30 Sekunden bis 2 Minuten, insbesondere bevorzugt ca. 50 bis 70 Sekunden und ganz besonders bevorzugt ca. 60 Sekunden.

Die Regelung des Lüfters kann - entsprechend der Stellung des inneren Hohlzylinders im äußeren Hohlzylinder - auch intervallweise erfolgen, bspw. kann die Drehzahl nach einer Stellungsänderung der Hohlzylinder am Anfang gering sein und derart geregelt werden, dass sie langsam ansteigt.

Die Regelung der Beduftung kann jedoch auch bedarfsorientiert erfolgen, wofür entsprechende Sensoren vorzusehen sind.

In einer weiteren Variante wird das Beduftungssystem bevorzugt in einem Intervall betrieb betrieben, bei dem in einem ersten Schritt in einem ersten Zeitintervall die Beduftung mit einem ersten Duftstoff und in einem zweiten Schritt in einem zweiten Zeitintervall die Beduftung mit einem zweiten vom ersten Duftstoff verschiedenen Duftstoff erfolgt. Zwischen dem ersten und zweiten Schritt liegt bevorzugt ein Zeitintervall ohne Beduftung. Vorteilhafterweise erfolgt der Betrieb des Beduftungssystems in sequentieller Abfolge der Beduftung mit verschiedenen Duftstoffen und Beduftungspausen programmgesteuert.

Im Folgenden wird die Erfindung anhand von Ausführungsbeispielen, teilweise unter Bezugnahme auf die Zeichnung, im Einzelnen erläutert. Es zeigen:
- Fig. 1: einen schematischen Schnitt quer durch ein erfindungsgemäßes Beduftungssystem gemäß dem ersten Ausführungsbeispiel,
- Fig. 2: einen schematischen Längsschnitt durch das Beduftungssystem von Fig. 1,
- Fig.3: einen Längsschnitt durch ein Beduftungssystem gemäß dem zweiten Ausführungsbeispiel,
- Fig. 4: einen Schnitt quer durch den inneren Zylinder des Beduftungssystems von Fig. 3,
- Fig. 5: eine Seitenansicht des inneren Zylinders von Fig. 4,
- Fig. 6: einen Schnitt quer durch ein Beduftungssystem gemäß dem dritten Ausführungsbeispiel,
- Fig. 7: einen Schnitt quer durch ein Beduftungssystem gemäß dem vierten Ausführungsbeispiel,
- Fig. 8: einen Schnitt quer durch ein Beduftungssystem gemäß dem fünften Ausführungsbeispiel, und
- Fig. 9: einen Schnitt quer durch ein Beduftungssystem gemäß dem sechsten Ausführungsbeispiel.

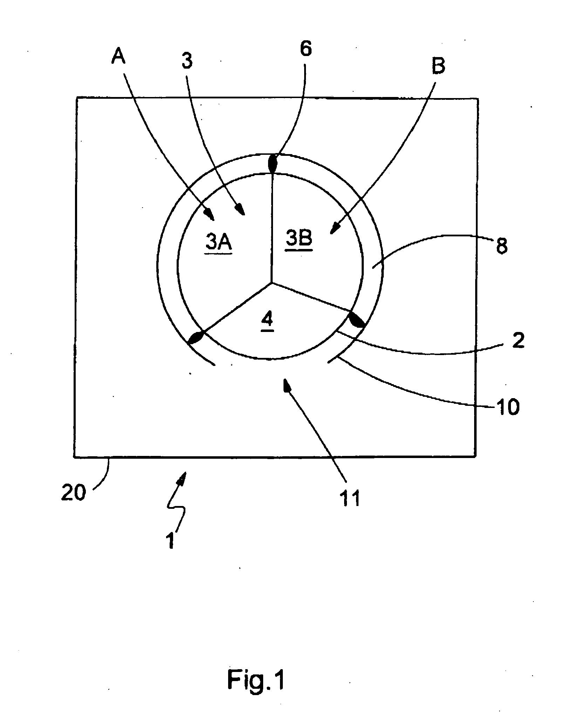

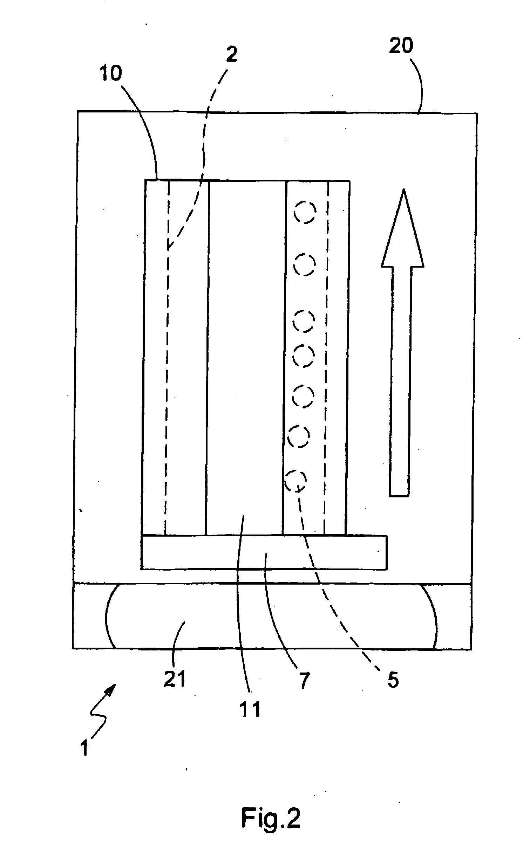

Ein zentral in einer Instrumententafel eines Kraftfahrzeugs angeordnetes Beduftungssystem 1 gemäß dem ersten, in den Figuren 1 und 2 dargestellten Ausführungsbeispiel weist ein erstes Bauteil 2 in Form eines inneren Hohlzylinders mit mehreren Durftreservoirs 3, vorliegend die mit einem ersten Duft A gefüllte Aufnahmekammer 3A und die mit einem zweiten Duft B gefüllte Aufnahmekammer 3B, und einer leeren, d.h. duftlosen Aufnahmekammer 4 auf, welche die Nullprobe bildet. Die einzelnen Aufnahmekammern 3A, 3B und 4 sind mittels Trennwänden voneinander getrennt, wobei vorliegend die Aufnahmekammern jeweils gleich groß sind und die Trennwände sich in der Längsachse des inneren Hohlzylinders treffen. Jeder der Aufnahmekammern weist mindestens eine Öffnung, vorliegend eine Mehrzahl von Bohrungen 5, auf, durch welchen der Duft aus der Aufnahmekammer austreten kann. Der innere Hohlzylinder ist oben und unten durch Wände verschlossen.

Der innere Hohlzylinder ist mittels eines Antriebs 7 (vorliegend ein elektrischer Schrittstellmotor) drehbar in einen eine Umschließung bildenden zweiten Bauteil 10, vorliegend einem äußeren Hohlzylinder, angeordnet, wobei ein Ringspalt 8 zwischen der Außenmantelfläche des inneren Hohlzylinders und der Innenmantelfläche des äußeren Hohlzylinders ausgebildet ist, der durch elastische Dichtelemente 6 unterbrochen ist, die an der Außenmantelfläche des inneren Hohlzylinders in Verlängerung der Trennwände, welche die Aufnahmekammern 3A, 3B und 4 unterteilen, angeordnet sind. Der äußere Hohlzylinder ist fest mit dem Gehäuse des Antriebs 7 verbunden, während der innere Hohlzylinder mit der Abtriebswelle desselben verbunden ist.

Der äußere Hohlzylinder weist einen sich in Richtung seiner Längsachse erstreckenden und über die gesamte Länge des äußeren Hohlzylinders ausgebildeten Schlitz 11 auf, der vorliegend einen Winkelbereich von ca. 50° einnimmt, also relativ breit ausgebildet ist.

Die gesamte Anordnung der Hohlzylinder 2 sowie des Elektromotors 7 ist innerhalb eines Gehäuses 20 angeordnet, welches der Luftführung vorbei am Schlitz 11 dient. Die Luft wird mit Hilfe eines Lüfters 21, welches benachbart dem Elektromotor 7 auf einer Seite des Gehäuses 20 angeordnet ist, vorbei am Schlitz 11 gefördert und verlässt das Gehäuse 20 auf der gegenüberliegenden Seite. Für die Ansaugung der Luft ist auf der Lüfterseite eine Lufteintrittsöffnung im Gehäuse 20 vorgesehen. Der Lüfter 21 wird mittels eines zweiten Elektromotors (nicht dargestellt) angetrieben. Alternativ ist jedoch auch eine temporäre Koppelung der Antriebe von Lüfter und innerem Hohlzylinder bei Bedarf einer Stellbewegung des inneren Hohlkörpers oder Hohlzylinders möglich, wofür zusätzlich ein Getriebe zur Änderung der Drehzahl für den Antrieb des inneren Hohlzylinders erforderlich ist.

Die Anbringung der Dichtelemente kann gegebenenfalls auch auf der Innenmantelfläche des äußeren Hohlzylinders erfolgen. Ebenso ist prinzipiell auch ein Drehen des äußeren Hohlzylinders um den inneren Hohlzylinder möglich, d.h. der innere Hohlzylinder ist ortsfest angebracht und der äußere Hohlzylinder dreh- und/oder einstellbar, bspw. mittels eines Elektromotors.

Der Betrieb des Beduftungssystems gemäß dem ersten Ausführungsbeispiel kann folgendermaßen erfolgen: Wird keine Beduftung benötigt, so wird die Nullprobe, d.h. die Reihe der Bohrungen 5 zur Aufnahmekammer 4 an den Schlitz 11 gedreht. Soll eine 50%-ige Beduftung, d.h. die Abgabe von etwas Duftstoff, vorliegend dem Duftstoff A, der in der Aufnahmekammer 3A enthalten ist, an den Fahrzeuginnenraum erfolgen, so wird der innere Hohlzylinder vom Elektromotor 7 in eine Winkelstellung gedreht, bei der sowohl die Bohrungen 5 zur Aufnahmekammer 4 über den Ringspalt 8 als auch die Bohrungen 5 zur Aufnahmekammer 3A mit dem vorliegend gewünschten Duftstoff über den Ringspalt 8 direkten Zugang zum Schlitz 11 haben, d.h. ohne Unterbrechung des Ringspalts 8 durch ein Dichtelement 6. Soll hingegen komplett mit dem Duftstoff A beduftet werden, so wird der innere Hohlzylinder weitergedreht, bis die Bohrungen 5 zur Aufnahmekammer 3A zentral und radial innerhalb des Schlitzes 11 angeordnet sind. Ferner ist ein Mischen der Düfte A und B möglich, wofür die Bohrungen 5 zu den Aufnahmekammern 3A und 3B direkt, d.h. ohne Unterbrechung durch ein Dichtelement 6, über den Ringspalt mit dem Schlitz 11 verbunden sind.

Die Beduftung selbst erfolgt vorliegend im impulsbetrieb, d.h. in definierten Zeitintervallen wird für eine vorgegebene Zeitspanne kein Duft abgegeben, d.h. die Nullprobe ist über die Bohrungen 5 fluchtend mit dem Schlitz 11 verbunden.

Vorliegend erfolgt bei geringen Außentemperaturen eine Beduftung mit dem Duftstoff A jeweils für zehn Sekunden und anschließend erfolgt keine Abgabe eines Duftstoffes für eine Zeitspanne von einer Minute, so dass eine Gewöhnung der Fahrzeuginsassen an den Duftstoff A vermieden wird, d.h. die Dosierung muss nicht gesteigert werden. Bei hohen Außentemperaturen erfolgt eine Beduftung mit dem Duftstoff B und bei "normalen" Temperaturen eine Beduftung mit einer Mischung der Duftstoffe A und B, wobei die Zeitintervalle gemäß dem vorliegenden Ausführungsbeispiel jeweils gleich sind.

Der Lüfter 21 sorgt dafür, dass der gewünschte Duftstoff oder die gewünschte Duftstoffmischung, deren Bohrungen 5 benachbart des Schlitzes 11 angeordnet sind, aus dem Gehäuse 20 ausgeblasen und an den Fahrzeuginnenraum abgegeben wird.

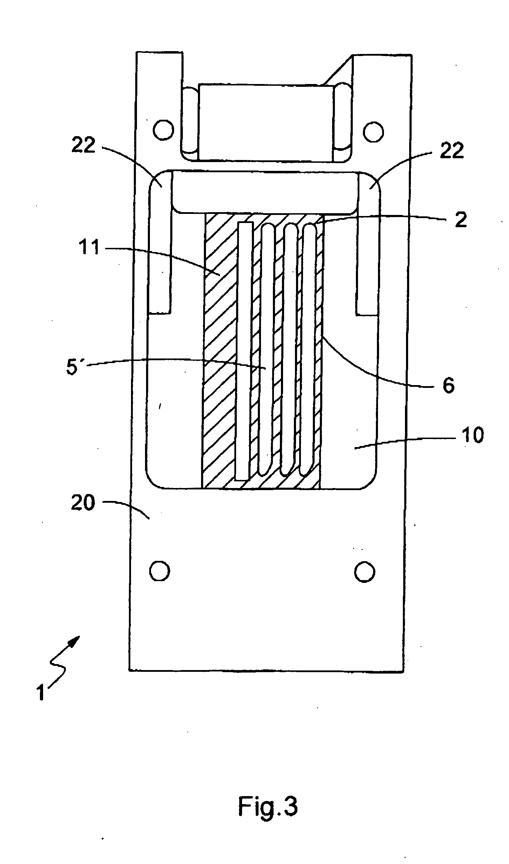

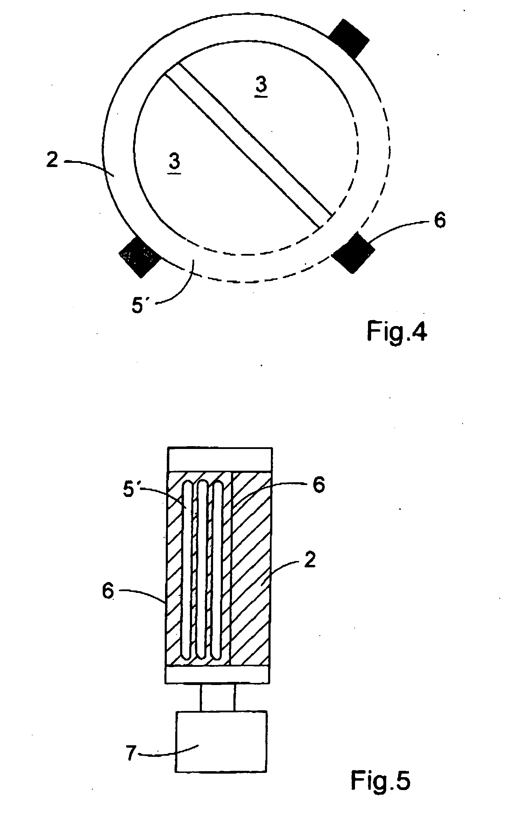

Gemäß dem in den Figuren 3 bis 5 dargestellten, zweiten Ausführungsbeispiel ist ein mittels eines Elektromotors 7, vorliegend eines Schrittstellmotors, drehbar gelagerter innerer Hohlzylinder (erstes Bauteil 2) mit zwei Duftreservoirs 3, die durch eine in Längsrichtung des inneren Hohlzylinders verlaufende Trennwand unterteilt sind, ausgebildet. Um ein Austreten von Duftstoff(en) aus den Duftreservoirs 3 zu ermöglichen, ist in den Mantelflächen des inneren Hohlzylinders eine Mehrzahl von sich in Längsrichtung über nahezu den gesamten inneren Hohlzylinder erstreckenden Schlitzen 5' vorgesehen. In Verlängerung der Trennwand sind jeweils außenseitig am inneren Hohlzylinder Dichtelemente 6 angeordnet, die außenseitig an der Innenmantelfläche eines äußeren hohlzylindrischen Bauteils (zweites Bauteil 10) anliegen und so ein Vermischen der Duftstoffe verhindern. Die Dichtelemente 6 sind vorliegend zwischen und außenseitig der Bereiche mit den Schlitzen 5' angeordnet. In der dargestellten Stellung des Inneren Hohlzylinders gelangt Duftstoff durch die Schlitze 5' durch den Schlitz bzw. die Öffnung 11, wobei die Schlitze 5' etwa die rechte Hälfte der Öffnung 11 einnehmen. Die linke Hälfte der Öffnung 11 im äußeren Hohlkörper wird durch den nicht geschlitzten Wandbereich des inneren Hohlzylinders verschlossen.

Im in Fig. 3 oben dargestellten Bereich sind bis nahe an die Mitte des äußeren Hohlzylinders reichende Öffnungen oder Kanäle 22 vorgesehen (nur schematisch angedeutet), die ein Ausströmen des gewünschten Duftstoffs bzw. der gewünschten Duftstoffe nach außen ermöglichen.

Entsprechend dem ersten Ausführungsbeispiel kann auch ein Lüfter (nicht dargestellt) vorgesehen sein, welcher für einen definierten Luftstrom vorbei an der Öffnung des äußeren Hohlzylinders sorgt.

Die Regelung kann sowohl getaktet, wie zuvor beim ersten Ausführungsbeispiel beschrieben, als auch in Abhängigkeit von Messwerten, ermittelt über entsprechende Duftsensoren, und in Abhängigkeit der ermittelten Konzentration und/oder Güte der Luft erfolgen.

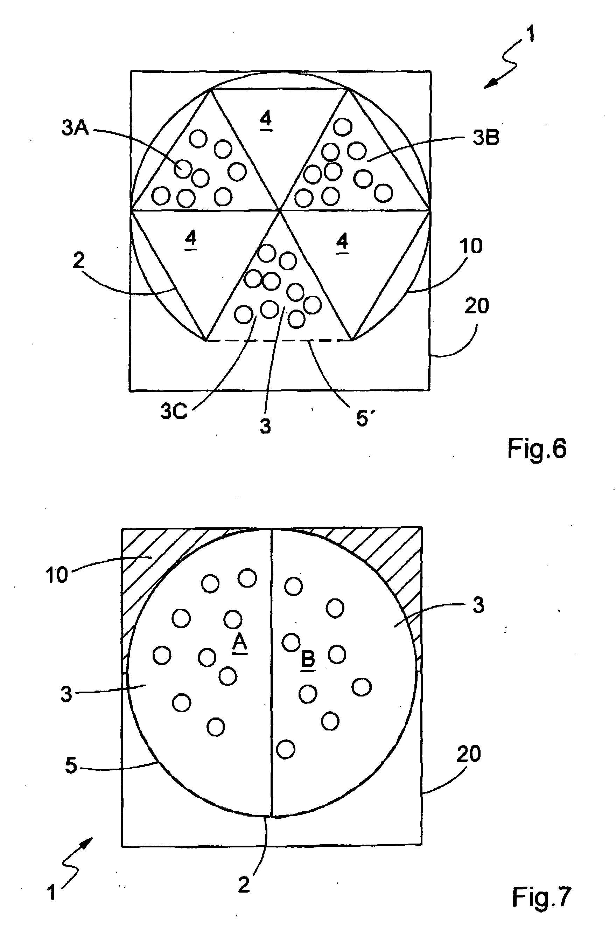

Fig. 6 zeigt ein drittes Ausführungsbeispiel eines Beduftungssystems 1, gemäß dem drei unterschiedliche Düfte A, B, C in einem inneren, prismenförmig ausgebildeten ersten Bauteil vorgesehen sind. Das innere, erste Bauteil 2, welches in seiner Funktion den zuvor beschriebenen inneren Hohlzylindern entspricht, weist hierbei einen gleichmäßig sechseckigen Querschnitt auf, in dem sechs Trennwände vorgesehen sind, so dass die einzelnen Aufnahmekammern 3A, 3B, 3C oder Duftreservoirs 3 sowie die jeweils zwischen zwei Duftreservoirs angeordneten duftlosen Aufnahmekammern 4 jeweils eine Querschnitt eines gleichseitigen Dreiecks haben. Die Außenfläche, die auch als Außenmantelfläche bezeichnet wird, weist im Bereich der Duftreservoirs 3, die gefüllt sind, eine Mehrzahl von sich in Längsrichtung erstreckenden Schlitzen 5' als Öffnungen auf.

Um das innere Bauteil 2 ist ein auf einer Seite geschlitzter und daher C-förmig ausgebildeter Hohlzylinder als zweites Bauteil 10 angeordnet, wobei der Innendurchmesser des Hohlzylinders dem maximalen Durchmesser des inneren Bauteils 2 entspricht. Dabei ist das innere Bauteil 2 mit Hilfe eines Elektromotors (nicht dargestellt) im zweiten, äußeren Bauteil 10 drehbar. Auf Grund der Außenkontur des inneren Bauteils 2 können zusätzliche Dichtelemente im Bereich der Kanten des inneren Bauteils 2 entfallen.

Die beiden Bauteile 2 und 10 sind in einem Gehäuse 20 mit vorliegend quadratischem Querschnitt angeordnet, welches zumindest auf der Seite, auf welcher der Schlitz angeordnet ist, von Luft durchströmbar ist, die - vorbeiströmend am Schlitz des zweiten, äußeren Bauteils 10 - den gewünschten Duftstoff des an den Schlitz angrenzenden Duftreservoirs 3 zum Fahrzeuginnenraum transportiert. Je nach Winkelstellung des inneren Bauteils 2 im äußeren Bauteil 10 wird mehr, weniger oder überhaupt kein Duftstoff freigesetzt. Die Regelung kann erfolgen, wie zuvor beschrieben, also beispielsweise im Intervallbetrieb oder bedarfsgeregelt.

Die Befüllung der einzelnen Aufnahmekammern erfolgt mittels Einschiebens von Patronen, die den entsprechenden, gewünschten Duftstoff enthalten, in Richtung der Drehachse des ersten Bauteils 2.

Dadurch, dass jeweils leere Aufnahmekammern 4 zwischen zwei gefüllten Aufnahmekammern 3A und 3B, 3B und 3C, 3C und 3A angeordnet sind, kann über Zwischenstellungen eine Verringerung der Beduftung ermöglicht werden, ohne dass ein unerwünschter Duftstoff beigemischt wird.

Durch eine geänderte Ausgestaltung der Bereiche, in denen die Schlitze angeordnet sind, und eine Verringerung der Schlitzbreite des zweiten, äußeren Bauteils 10 können auch alle Aufnahmekammern gefüllt werden, so dass bei der vorliegenden Anordnung der Trennwände maximal sechs Duftreservoirs möglich sind.

Alternativ wäre als prismatisches Bauteil beispielsweise auch ein Achteck möglich, welches bei der vorstehenden Ausgestaltung 4 bzw. maximal 8 Duftreservoirs aufweisen kann. Beliebige andere prismatische Bauteile, vorzugsweise mit Querschnitten von gleichseitigen Vielecken sind möglich.

Fig. 7 zeigt ein viertes Ausführungsbeispiel eines Beduftungssystems 1. Hierbei ist vorliegend das innere, die Duftstoffe (A und B) enthaltende Bauteil 2 als Hohlzylinder ausgebildet, wobei eine in Längsrichtung mittig verlaufende Trennwand den Hohlzylinder in zwei gleich große Duftreservoirs 3 teilt. Der Hohlzylinder ist über die Hälfte seines Umfangs mit einer Perforation, gebildet durch eine Vielzahl von Bohrungen 5, versehen, wobei der perforierte Bereich sich jeweils über die Hälfte der beiden Duftreservoirs 3 erstreckt.

Das innere, erste Bauteil 2 wird über die Hälfte seines Umfangs von einem zweiten, C-förmig ausgebildeten Bauteil 10 umgriffen, welches über diesen Bereich des Umfangs das erste Bauteil 2 abdeckt, also ggf. hier angeordnete Öffnungen, d.h. vorliegend Bohrungen 5, verschließt.

Die beiden Bauteile 2 und 10 sind in einem Gehäuse 20 mit rechteckförmigem Querschnitt angeordnet, so dass ein Spalt auf der dem zweiten Bauteil 10 gegenüberliegenden Seite frei ist, durch Luft, gefördert durch einen Lüfter, strömen und ggf. den Duftstoff mitnehmen kann.

Auch im vorliegenden Ausführungsbeispiel dreht sich das erste, innere Bauteil 2 im äußeren, zweiten Bauteil 10, um den Zustand der Beduftung zu regeln. Die Regelung kann wie zuvor erwähnt erfolgen.

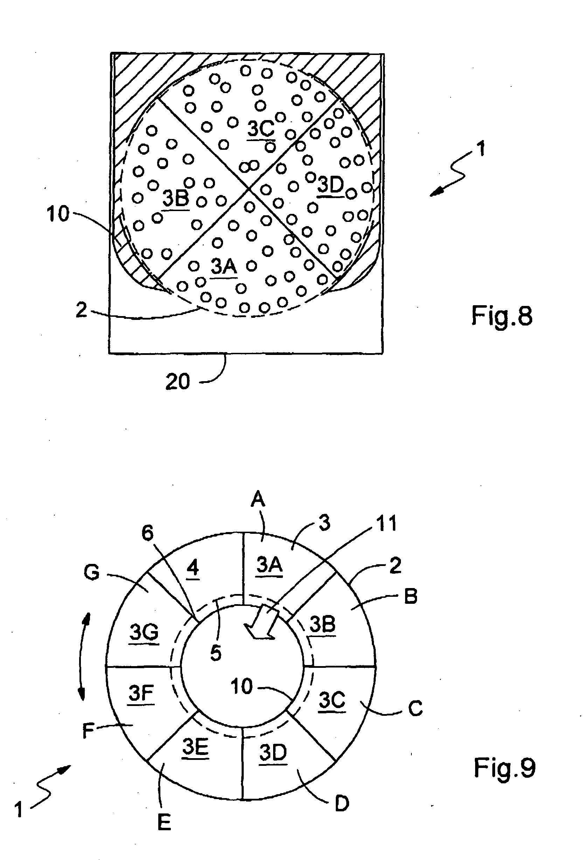

Gemäß dem fünften, in Fig. 8 dargestellten Ausführungsbeispiel, welches dem zuvor beschriebenen vierten Ausführungsbeispiel im Prinzip entspricht, sind im inneren Bauteil 2, welches als Hohlzylinder ausgebildet ist, vier Aufnahmekammern 3A, 3B, 3C und 3D vorgesehen, die durch Trennwände voneinander getrennt sind und jeweils eine auswechselbare Duftpatrone enthalten. Die Trennwände schneiden die Drehachse des inneren Bauteils und stehen senkrecht zueinander. Das innere Bauteil 2 wird von einem äußeren Bauteil 10 C-förmig um mehr als 270° umgriffen, so dass jeweils nur ein Teilbereich der Mantelfläche eines oder zweier Segmente freigegeben ist, In der Mantelfläche des inneren Bauteils 2 sind bei jedem Duftreservoir 3 Schlitze 5' ausgebildet, durch welche der entsprechende Duftstoff bei Bedarf nach außen gelangen und einem vorbeiströmenden Luftstrom zugeführt werden kann.

Auch bei diesem Ausführungsbeispiel dreht sich das erste, innere Bauteil 2 im äußeren, zweiten Bauteil 10, um den Zustand der Beduftung zu regeln. Die Regelung kann wiederum wie zuvor erwähnt erfolgen.

Das sechste Ausführungsbeispiel unterscheidet sich deutlich von den zuvor beschriebenen Ausführungsbeispielen. Hierbei ist das erste Bauteil 2 dieses Beduftungssystems 1 als drehbarer Ring mit einer Mehrzahl von (vorliegend acht) Aufnahmekammern 3A-3G und 4 ausgebildet, die voneinander mittels Trennwänden getrennt sind, die jeweils in Ebenen angeordnet sind, welche sich in der Drehachse schneiden. Die Innenmantelfläche des ersten Bauteils 2 ist mit einer Vielzahl von Bohrungen 5 versehen, über welche die Duftstoffe A-G austreten können.

Im Inneren des ersten, drehbaren Bauteils 2 ist ein zweites Bauteil 10 in Form eines inneren Hohlzylinders mit einer Öffnung in Form eines Schlitzes 11 angeordnet. Der Außendurchmesser des zweiten Bauteils 10 ist etwas kleiner als der Innendurchmesser des ersten Bauteils, so dass zwischen den beiden Bauteilen Dichtelemente 6 jeweils in Verlängerung der Trennwände der Aufnahmekammern angeordnet sind.

Der Luftstrom, der den entsprechenden, gewünschten Duft aufnehmen soll, strömt vorliegend im Inneren des zweiten, inneren Bauteils 10, so dass dieses Bauteil zudem einen Luftkanal bildet. Die Regelung der Intensität der Luft erfolgt vorliegend über die Drehzahl des entsprechenden Lüfters (nicht dargestellt) und im Falle der Duftstoffe A und G zusätzlich über die entsprechende Relativposition der Bohrungen 5 zum Schlitz 11, jedoch ist alternativ auch eine Ausgestaltung des ersten Bauteils mit einer Mehrzahl von leeren Aufnahmekammern 4 möglich.

Die Betätigung des ersten Bauteils kann bspw. über eine Verzahnung, die an der Außenseite vorgesehen ist, erfolgen.

Gemäß einer alternativen Ausführungsform ist das zweite Bauteil 10 im ersten Bauteil 2 verdrehbar angeordnet, so dass sich die Position des Schlitzes 11 ändert, die der Aufnahmekammern jedoch nicht.

Der Zustand der Nichtbeduftung kann gegebenenfalls auch dadurch erreicht werden, dass der Lüfter steht, so dass keine Luft vorbei an der Öffnung des abdeckenden Bauteils strömt und den Duftstoff transportiert. Die Konzentration des Duftstoffs wird durch die Drehzahl des Lüfters geregelt. Dabei kann der Betrieb des Lüfters beispielsweise derart erfolgen, dass auf zehn Sekunden Lüfterbetrieb sechzig Sekunden Pause folgen. Neben einer Intervallregelung ist auch eine bedarfsgerechte Regelung in Abhängigkeit von ermittelten Messwerten (Duftkonzentration, Temperatur, Fahrtgeschwindigkeit o.ä.) möglich.

Die Duftstoffe, beispielsweise vorliegend als Granulate, können direkt in den Duftreservoirs aufgenommen sein, jedoch ist es vorteilhaft, wenn die Duftstoffe in auswechselbaren, vorzugsweise wiederbefüllbaren Patronen enthalten sind, die in die entsprechenden Aufnahmekammern einschiebbar sind.

Die Relativbewegung zwischen den beiden Bauteilen muss nicht notwendigerweise nur durch eines derselben erfolgen, sondern es können sich auch beide Bauteile bewegen, bspw. in entgegengesetzte Richtungen oder in gleicher Richtung aber mit unterschiedlichen Geschwindigkeiten.

Ferner muss es sich bei der Relativbewegung nicht notwendigerweise um eine Drehbewegung handeln, sondern es sind vielmehr auch Längsverschiebungen, Schraubbewegungen oder beliebige Taumelbewegungen möglich, welche in einzelnen Stellungen der Bauteile zueinander zwischen mindestens zwei Öffnungen in denselben einen freien Durchgang ermöglicht, so dass der Duftstoff (oder die Duftstoffe) austreten können. Insbesondere im Falle einer Drehbewegung, die mit einer Längsbewegung kombiniert werden kann, können neben der segmentartigen Unterteilung auch zwei oder mehr Segmentringe o.ä. von Duftreservoirs in Längsrichtung nebeneinander angeordnet sein. Die Öffnung im verschließenden Bauteil ist hierbei bevorzugt derart ausgebildet, dass sie sich maximal über die Länge eines Duftreservoirs in Längsrichtung und über eine Breite im Bereich der Öffnungen eines Duftreservoirs in Umfangsrichtung erstreckt.

Die Unterteilung der Duftreservoirs muss nicht notwendigerweise durch Trennwände erfolgen, welche (zumindest in ihrer Verlängerung) die Drehachse schneiden, sondern sie können auch auf beliebige andere Weise unterteilt sein. Ferner können auch Trennwände quer, insbesondere senkrecht zur Drehachse bzw. Bewegungsrichtung vorgesehen sein, so dass über die Länge der Bauteile mehrere Duftreservoirs vorgesehen sein können.

Gemäß den zuvor beschriebenen Ausführungsbeispielen erfolgt die Erzeugung eines Luftstroms durch einen Lüfter, der unabhängig vom Gebläse einer Klimaanlage o.ä. betätigbar ist, so dass derartige Beduftungssysteme in Alleinstellung betrieben werden können, also insbesondere nicht notwendigerweise fest in ein Kraftfahrzeug integriert sein müssen, was jedoch bei dieser Ausgestaltung auch möglich ist. Alternativ kann der Luftstrom, der an der Öffnung des zweiten Bauteils vorbeiströmt und den Duftstoff oder die Duftstoffe dem Fahrzeuginnenraum zuführt, auch direkt vom durch das Gebläse der Klimaanlage o.ä. geförderten Luftstrom abgezweigt werden. Die Abzweigung kann bevorzugt direkt nach dem Verdampfer erfolgen, so dass die Luft gekühlt ist und erfrischender wirkt. Eine Abzweigung nach dem Heizer ist ebenso möglich wie die Abzweigung aus einem Umluftkanal. Bevorzugt kann hierfür ein flexibler Schlauch oder ein Kanal aus Kunststoff vorgesehen sein.

## Patentansprüche

1. Beduftungssystem (1) insbesondere für ein Kraftfahrzeug, mit zumindest einem ersten Bauteil (2) und zumindest einem zweiten Bauteil (10), welches das zumindest erste Bauteil (2) im Wesentlichen umgreift oder von diesem umgriffen wird, wobei
das zumindest eine erste (2) und das zumindest eine zweite (10) Bauteil jeweils eine prismatische Innen- beziehungsweise Außenmantelfläche aufweisen, die auch unterbrochen ausgebildet sein kann, und die Bauteile (2, 10) relativ zueinander bewegbar sind, wobei
in einem der Bauteile (2) mindestens zwei Aufnahmekammem (3A, 3B, 3C, 3D, 3E, 3F, 3G, 4) vorgesehen sind, in welchen mindestens ein Duftstoff (A, B, C, D, E, F, G) enthalten ist, und
durch das zumindest eine, erste Bauteil (2) und das zumindest eine, zweite Bauteil (10) in Ahängigkeit der Position zueinander mindestens eine Verbindung zur Umgebung (11) freigeben ist, über welche bei Bedarf Duftstoff (A, B, C, D, E, F, G) nach außen aus der oder den Aufnahmekammern (3A, 3B, 3C, 3D, 3E, 3F, 3G) abgegeben werden kann, wobei
die Aufnahmekammem (3A, 3B, 3C, 3D, 3E, 3F, 3G, 4) durch zumindest eine in Längsrichtung des Bauteils (2) verlaufende Trennwand voneinander getrennt sind, und
die Verbindung zur Umgebung vorgesehen ist über zumindest eine in einer prismatischen Mantelfläche des Bauteils (2), in welchem die Aufnahmekammem (3A, 3B, 3C, 3D, 3E, 3F, 3G, 4) für den oder die Duftstoffe angeordnet sind, ausgebildete Öffnung (5) und mindestens eine Öffnung (5) in der gegenüberliegend angeordneten Mantelfläche des anderen Bauteils (10), **dadurch gekennzeichnet, dass** das erste Bauteil (2) und das zweite Bauteil (10) in einem Gehäuse (20) angeordnet sind, in oder neben welchem ferner ein Lüfter (21) zur Erzeugung eines Luftstroms vorbei an der mindestens einen Öffnung des zweiten Bauteils (10) angeordnet ist, wobei der Lüfter (21) unabhängig vom Gebläse einer Klimaanlage betätigbar ist.

2. Beduftungssystem nach Anspruch 1, **dadurch gekennzeichnet, dass** mindestens eine prismatische Mantelfläche des mindestens einen, ersten Bauteils (s) und/oder des mindestens einen, zweiten Bauteils (10) kreiszylindrisch ist.

3. Beduftungssystem nach Anspruch 2, **dadurch gekennzeichnet, dass** die prismatische Mantelflächen des ersten Bauteils (2) und die prismatische Mantelflächen des zweiten Bauteils (10) jeweils kreiszylindrisch ausgebildet und konzentrisch zueinander angeordnet sind.

4. Beduftungssystem nach einem der vorhergehenden Ansprüche, **dadurch gekennzeichnet, dass** die Aufnahmekammem (3A, 3B, 4) in dem Bauteil angeordnet sind, welches vom anderen Bauteil umgriffen wird, und dass die Öffnungen der Aufnahmekammem (3A, 3B, 4) in der Außenmantelfläche des Bauteils angeordnet sind.

5. Beduftungssystem nach einem der vorhergehenden Ansprüche, **dadurch gekennzeichnet, dass** das Bauteil, in welchem die Aufnahmekammern (3A, 3B, 4) angeordnet sind, und/oder das andere Bauteil drehbar angeordnet ist.

6. Beduftungssystem nach einem der vorhergehenden Ansprüche, **dadurch gekennzeichnet, dass** die Trennwände zwischen den Aufnahmekammem (3A, 38, 3C, 3D, 3E, 3F, 3G, 4) in radialer Richtung bezüglich der Drehachse des oder der Bauteile angeordnet sind, um die mindestens eines der Bauteile drehbar ist.

7. Beduftungssystem nach einem der vorhergehenden Ansprüche, **dadurch gekennzeichnet, dass** das Bauteil, in welchem die Aufnahmekammem (3A, 3B, 4) angeordnet sind, und/oder das andere Bauteil in axialer Richtung verschiebbar angeordnet ist.

8. Beduftungssystem nach einem der vorhergehenden Ansprüche, **dadurch gekennzeichnet, dass** im ersten Bauteil (2) mit den Aufnahmekammern (3A, 3B, 3C, 3D, 3E, 3F, 3G, 4) für Duftstoff mindestens eine Aufnahmekammer (4) ohne einen Duftstoff vorgesehen ist, insbesondere jede zweite Aufnahmekammer eine Aufnahmekammer (4) ohne Duftstoff ist.

9. Beduftungssystem nach einem der vorhergehenden Ansprüche, **dadurch gekennzeichnet, dass** zwischen den Bauteilen (2, 10) Dichtelemente (6), insbesondere in Verlängerung der Trennwände, angeordnet sind.

10. Beduftungssystem nach einem der vorhergehenden Ansprüche, **dadurch gekennzeichnet, dass** die Aufnahmekammem (3A, 3B, 3C, 3D, 3E, 3F, 3G, 4) jeweils gleiche Volumen aufweisen.

## Claims

1. A fragrance system (1), in particular for a motor vehicle, comprising at least one first component (2) and at least one second component (10) which substantially encloses the at least one first component (2) or is enclosed thereby, wherein
the at least one first component (2) and the at least one second component (10) each have a prismatic inner and outer jacket surface that can also have an interrupted design, and the components (2, 10) are moveable relative to each other, wherein
in one of the components (2) at least two receiving chambers (3A, 3B, 3C, 3D, 3E, 3F, 3G, 4) are provided, in which at least one fragrance (A, B, C, D, E, F, G) is contained, and
at least one connection to the surroundings (11) is opened by the at least one first component (2) and the at least one second component (10) depending on their position relative to each other, via which connection fragrance (A, B, C, D, E, F, G) can be dispensed, as necessary, outwardly out of the receiving chamber(s) (3A, 3B, 3C, 3D, 3E, 3F, 3G), wherein
the receiving chambers (3A, 3B, 3C, 3D, 3E, 3F, 3G, 4) are separated from each other by at least one partition wall that extends in the longitudinal direction of the component (2), and
the connection to the surroundings is provided via at least one opening (5) in a prismatic jacket surface of the component (2), in which the receiving chambers (3A, 3B, 3C, 3D, 3E, 3F, 3G, 4) for the fragrance(s) are disposed, and at least one opening (5) in the diametrically opposed jacket surface of the other component (10), **characterized in that** the first component (2) and the second component (10) are disposed in a housing (20) in which, or next to which, a fan (21) is likewise disposed to create an air flow past the at least one opening of the second component (10), it being possible to actuate the fan (21) independently of the blower of an air-conditioning system.

2. The fragrance system according to claim 1, **characterized in that** at least one prismatic jacket surface of the at least one first component (2) and/or the at least one second component (10) is circular-cylindrical.

3. The fragrance system according to claim 2, **characterized in that** the prismatic jacket surfaces of the first component (2) and the prismatic jacket surfaces of the second component (10) are each circular-cylindrical and are disposed concentrically to each other.

4. The fragrance system according to one of the preceding claims, **characterized in that** the receiving chambers (3A, 3B, 4) are disposed in the component that is enclosed by the other component, and the openings in the receiving chambers (3A, 3B, 4) are disposed in the outer jacket surface of the component.

5. The fragrance system according to one of the preceding claims, **characterized in that** the component in which the receiving chambers (3A, 3B, 4) are disposed, and/or the other component is rotatably disposed.

6. The fragrance system according to one of the preceding claims, **characterized in that** the partition walls between the receiving chambers (3A, 3B, 3C, 3D, 3E, 3F, 3G, 4) are disposed in the radial direction relative to the rotational axis of the component(s), about which at least one of the components can rotate.

7. The fragrance system according to one of the preceding claims, **characterized in that** the component in which the receiving chambers (3A, 3B, 4) are disposed, and/or the other component is disposed such that it is displaceable in the axial direction.

8. The fragrance system according to one of the preceding claims, **characterized in that** at least one receiving chamber (4) in the first component (2) having the receiving chambers (3A, 3B, 3C, 3D, 3E, 3F, 3G, 4) for fragrance does not contain fragrance, in particular every second receiving chamber is a receiving chamber (4) without fragrance.

9. The fragrance system according to one of the preceding claims, **characterized in that** sealing elements (6), in particular as extensions of the partition walls, are disposed between the components (2, 10).

10. The fragrance system according to one of the preceding claims, **characterized in that** the receiving chambers (3A, 3B, 3C, 3D, 3E, 3F, 3G, 4) all have the same volume.

## Revendications

1. Système (1) servant à parfumer, en particulier pour un véhicule automobile, comprenant au moins un premier composant (2) et au moins un deuxième composant (10) qui entoure pratiquement le premier composant (2) au moins au nombre de un, ou bien est entouré par celui-ci,
où le premier composant (2) au moins au nombre de un et le deuxième composant (10) au moins au nombre de un présentent respectivement une surface latérale intérieure ou extérieure prismatique qui peut être configurée également de manière discontinue, et les composants (2, 10) peuvent être mobiles l'un par rapport à l'autre, où il est prévu, dans l'un des composants (2), au moins deux chambres réceptrices (3A, 3B, 3C, 3D, 3E, 3F, 3G, 4) dans lesquelles est contenue au moins une matière odorante (A, B, C, D, E, F, G), et
au moins un passage (11) par rapport au milieu environnant est dégagé par le premier composant (2) au moins au nombre de un et par le deuxième composant (10) au moins au nombre de un, en fonction de la position de l'un par rapport à l'autre, passage par lequel, en cas de besoin, de la matière odorante (A, B, C, D, E, F, G) peut être fournie vers l'extérieur, à partir de la chambre ou des chambres réceptrice(s) (3A, 3B, 3C, 3D, 3E, 3F, 3G), où les chambres réceptrices (3A, 3B, 3C, 3D, 3E, 3F, 3G, 4) sont séparées les unes des autres par au moins une paroi de séparation s'étendant dans le sens de la longueur du composant (2), et
le passage par rapport au milieu environnant est prévu en étant formé par au moins une ouverture (5) configurée dans une surface latérale prismatique du composant (2) dans lequel sont disposées les chambres réceptrices (3A, 3B, 3C, 3D, 3E, 3F, 3G, 4) pour la ou les matière(s) odorante(s), et par au moins une ouverture (5) dans la surface latérale - faisant face à ladite surface prismatique - de l'autre composant (10),
**caractérisé en ce que** le premier composant (2) et le deuxième composant (10) sont disposés dans un boitier (20) dans lequel ou à proximité duquel est disposé en outre un ventilateur (21) servant à la production d'un flux d'air passant devant l'ouverture, au moins au nombre de un, du deuxième composant (10), où le ventilateur (21) peut être actionné indépendamment de la soufflante d'un système de climatisation.

2. Système servant à parfumer selon la revendication 1, **caractérisé en ce qu'**au moins une surface latérale prismatique du premier composant au moins au nombre de un et / ou du deuxième composant (10) au moins au nombre de un est de forme cylindrique circulaire.

3. Système servant à parfumer selon la revendication 2, **caractérisé en ce que** les surfaces latérales prismatiques du premier composant (2) et les surfaces latérales prismatiques du deuxième composant (10) sont configurées en étant à chaque fois de forme cylindrique circulaire et disposées de façon concentrique les unes par rapport au autres.

4. Système servant à parfumer selon l'une quelconque des revendications précédentes, **caractérisé en ce que** les chambres réceptrices (3A, 3B, 4) sont disposées dans le composant qui est entouré par l'autre composant, et **en ce que** les ouvertures des chambres réceptrices (3A, 3B, 4) sont disposées dans la surface latérale extérieure du composant.

5. Système servant à parfumer selon l'une quelconque des revendications précédentes, **caractérisé en ce que** le composant dans lequel sont disposées les chambres réceptrices (3A, 3B, 4), et / ou l'autre composant, sont disposés de façon rotative.

6. Système servant à parfumer selon l'une quelconque des revendications précédentes, **caractérisé en ce que** les parois de séparation sont disposées entre les chambres réceptrices (3A, 3B, 3C, 3D, 3E, 3F, 3G, 4), dans le sens radial par rapport à l'axe de rotation du ou des composant(s), axe de rotation autour duquel est monté en rotation au moins l'un des composants.

7. Système servant à parfumer selon l'une quelconque des revendications précédentes, **caractérisé en ce que** le composant dans lequel sont disposées les chambres réceptrices (3A, 3B, 4), et / ou l'autre composant, sont disposés en pouvant coulisser dans le sens axial.

8. Système servant à parfumer selon l'une quelconque des revendications précédentes, **caractérisé en ce que**, dans le premier composant (2) comportant les chambres réceptrices (3A, 3B, 3C, 3D, 3E, 3F, 3G, 4) pour la matière odorante, il est prévu au moins une chambre réceptrice (4) sans matière odorante, en particulier chaque deuxième chambre réceptrice est une chambre réceptrice (4) sans matière odorante.

9. Système servant à parfumer selon l'une quelconque des revendications précédentes, **caractérisé en ce que** des éléments d'étanchéité (6) sont disposés entre les composants (2, 10), en particulier dans le prolongement des parois de séparation.

10. Système servant à parfumer selon l'une quelconque des revendications précédentes, **caractérisé en ce que** les chambres réceptrices (3A, 3B, 3C, 3D, 3E, 3F, 3G, 4) présentent à chaque fois des volumes identiques.
